# EUROPEAN PATENT SPECIFICATION

(11) **EP 4 125 782 B1**
(45) Date of publication and mention of the grant of the patent: **20.03.2024**
(21) Application number: 21712554.1
(22) Date of filing: 22.03.2021
(51) Int. Cl.: A61K 8/23, A61K 8/27, A61K 8/49, A61Q 5/00, A61Q 17/00

(54) **METHOD OF TREATING THE SCALP**
VERFAHREN ZUR BEHANDLUNG DER KOPFHAUT
PROCÉDÉ DE TRAITEMENT DU CUIR CHEVELU

(30) Priority: 31.03.2020 EP 20167076
(43) Date of publication of application: 08.02.2023
(73) Proprietor: Unilever IP Holdings B.V., 3013 AL Rotterdam (NL); Unilever Global IP Limited, Wirral, Merseyside CH62 4ZD (GB)
(72) Inventor: BHOGAL, Ranjit, Kaur, Sharnbrook Bedfordshire MK44 1LQ (GB); JENKINS, Gail, Sharnbrook Bedfordshire MK44 1LQ (GB); SAWICKA, Magdalena, Sharnbrook Bedfordshire MK44 1LQ (GB)
(74) Representative: Tansley, Sally Elizabeth
(86) International application number: PCT/EP2021/057314
(87) International publication number: WO 2021/197908

(56) References cited:
- EP-A1- 3 213 753
- WO-A1-2004/035015
- WO-A1-2012/022553
- WO-A1-2014/180600
- WO-A1-2016/012973
- WO-A1-2016/157073
- FR-A1- 2 908 045
- FR-B1- 2 908 045

## Description

### Field of the Invention

The present invention relates to a method of strengthening the skin barrier. The skin barrier is preferably the scalp.

### Background of the Invention

Dandruff is a common skin disorder manifesting by the shedding of clumps of dead skin cells which are yellow or white, giving an aesthetically displeasing appearance during daily life. Therefore, dandruff refers to the generation of skin surface flake or the flake itself. The finding that dandruff is fundamentally a Malassezia-based disorder remains prevalent. Consequently, anti-fungal actives such as piroctone olamine aiming to control yeast of the genus Malassezia remain as conventional antidandruff agents.

Dandruff-prone individuals have weaker epidermal barrier, and scalp more susceptible to external stresses (microbes, surfactants). It has been found that improvement of the skin barrier, can mitigate the formation of dandruff.

US 2014/106016 describes the cosmetic use of an active agent derived from a microorganism belonging to the *Vitreoscilla* sp. genus (in particular the species: *Uitreoscilla filiformis)* as an active agent for preventing and/or treating dandruff conditions of the scalp, including dandruff conditions associated with a proliferation of pathogenic microorganisms on the scalp and/or an imbalance of the scalp ecoflora.

WO2013/010706 describes rapidly effective high-performance anti-dandruff products which are gentle to the scalp and to the hair, and which do not impair the combability, shine, or softness of the hair, containing 0.0001-5% epsilon-poly-L-lysine and 0.01-10% antidandruff agent selected from climbazole, zinc pyrithione, piroctone olamine, selenium sulphate and salicylic acid, or mixtures therefore.

WO 2014/180600 describes anti-dandruff formulations for the scalp functioning by increasing the skin barrier. The active agent is the azole compound climbazole (anti-fungal). The formulations lead to the up-regulation of expression of late cornified envelope proteins 3, small proline rich proteins and corneodesmosin (inter alia gene transcripts). This are all genes involved in cornified envelope formation, corneodesmosome formation and tight junction formation. Formulations further comprise anionic surfactant and/or conditioner.

WO 2012/022553 describes anti-dandruff formulations for the scalp functioning by increasing the skin barrier. The active agent is zinc pyrithione (anti-fungal) and an effective increase in barrier function was demonstrated. Formulations further comprise anionic surfactant and/or conditioner.

It is therefore an objective of the present invention to provide a composition that can enhance the health and strength of the skin/epidermal barrier.

It is also an objective of the present invention to provide a method for enhancing the health and strength of the skin/epidermal barrier. In an embodiment, this method is a cosmetic method.

### Description of the Invention

In a first aspect of the present invention, there is provided a non-therapeutic method of strengthening a skin barrier by application of a composition comprising at least one mRNA biomarker and/or mRNA biomarker group enhancement compound in which the mRNA biomarker/mRNA biomarker group is involved in cornified envelope formation, wherein the composition comprises a piroctone compound.

A second aspect of the invention relates to a composition comprising at least one mRNA biomarker and/or mRNA biomarker group enhancement compound in which the gene/gene group is involved in cornified envelope formation for use as a medicament in strengthening a skin barrier, wherein the composition comprises a piroctone compound.

A third aspect of the invention relates to the cosmetic use for strengthening the skin barrier of a composition comprising at least one mRNA biomarker and/or mRNA biomarker group enhancement compound in which the gene/gene compound is involved in cornified envelope formation, wherein the composition comprises a piroctone compound.

At least one mRNA biomarker and/or mRNA biomarker group enhancement compound in which the mRNA biomarker/mRNA biomarker group is involved in cornified envelope formation is a piroctone compound; conveniently it is piroctone olamine.

Herein, cosmetic use' should be understood to mean non-therapeutic use.

In an embodiment, the invention comprises a method of strengthening a skin barrier by application of a composition comprising a piroctone compound, preferably at a level of 0.3 to 1.0 wt% of the composition; conveniently the piroctone compound is piroctone olamine.
mRNA stands for messenger RNA.

The skin according to the present invention is preferably human scalp, more preferably healthy human scalp. By 'healthy human scalp' herein is meant the scalp wherein dandruff does not present.

For the avoidance of doubt, any feature of one aspect of the present invention may be utilised in any other aspect of the invention. Any feature described as 'preferred' should be understood to be particularly preferred in combination with a further preferred feature or features. Herein, any feature of a particular embodiment may be utilized in any other embodiment of the invention. The word 'comprising' is intended to mean 'including' but not necessarily 'consisting of or 'composed of. In other words, the listed steps or options need not be exhaustive. The examples given in the description below are intended to clarify the invention. All percentages are weight/weight percentages unless otherwise indicated.

Numerical ranges expressed in the format 'from x to y' are understood to include x and y, unless specified otherwise. When for a specific feature multiple preferred ranges are described in the format 'from x to y', it is understood that all ranges combining the different endpoints are also contemplated.

In the context of the present invention enhancement means a change in level of biomarker when compared to a baseline level of biomarker.

### Detailed Description of the Invention

The epidermal barrier resides in the uppermost layers of the epidermis stratum corneum and stratum granulosum, tight junctions in the stratum granulosum are cell-cell seals that limit the movements of molecules across the epidermis and hence contribute to barrier function.

The main functions of the epidermal barrier are protection from external environment and prevention of water loss.

The present invention relates to a non-therapeutic method of strengthening the skin barrier, preferably the skin barrier of the scalp by application of a composition comprising at least one mRNA biomarker enhancement compound in which the mRNA biomarker group is involved in cornified envelope formation, wherein the composition comprises a piroctone compound.

Preferably the mRNA biomarker group is further involved in comeodesmosome formation and tight junction formation.

It is thought that the treatment to restore and/or strengthen the epidermal barrier can reduce severity of dandruff, or generally improve a dry and/or itchy scalp condition.

Preferably the mRNA biomarkers are selected from the group consisting of Desmocolin 2, Desmoglein 4, Plakophilin 1, Claudin 4, Claudin 7, Claudin 17, Claudin 25, Late cornified envelope 3A, Late cornified envelope 3C, Late cornified envelope 3B, Small proline-rich protein 2D, Claudin 1, Zona Occludens 2 and Involucrin , more preferably Desmocolin 2, Desmoglein 4, Plakophilin 1, Claudin 4, Claudin 17, Claudin 2,5 Late cornified envelope 3A, Late cornified envelope 3C, most preferably Desmocolin 2, Desmoglein 4, Claudin 17, Claudin 25, Late cornified envelope 3A and Late cornified envelope 3C.

Compositions for the methods and uses of the invention comprise a mRNA biomarker/mRNA biomarker group enhancement compound. The mRNA biomarker/mRNA biomarker group enhancement compound is a piroctone compound preferably piroctone olamine.

Piroctone Olamine is an olamine salt of the hydroxamic acid derivative piroctone. It is commonly known as piroctone ethanolamine with the trade name Octopirox^{®}.

The piroctone olamine according to the present invention is a 1:1 compound of 1-hydroxy-4-methyl-6-(2,4,4-trimethylpentyl)-2(1*H*)-pyridinone with 2-aminoethanol and is also designated 1-hydroxy-4-methyl-6-(2,4,4-trimethylpentyl)-2(1*H*) pyridinone monoethanolamine salt. The CAS number is 68890-66-4 and the compound has the general formula (I) as below:

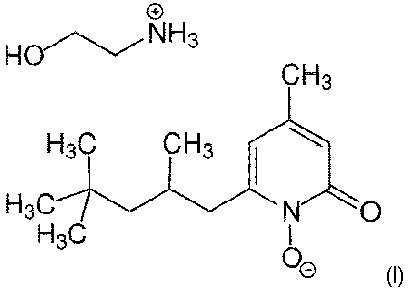

A piroctone compound may be comprised in the cosmetic composition from 0.01 to 5.0 %, by weight of the total composition, preferably from 0.05 to 2.5 wt%, more preferably from 0.1 to 1.5 wt%, most preferably from 0.3 to 1.0 wt%.

Piroctone olamine may be comprised in the cosmetic composition from 0.01 to 5.0 %, by weight of the total composition, preferably from 0.05 to 2.5 wt%, more preferably from 0.1 to 1.5 wt%, most preferably from 0.3 to 1.0 wt%.

### Formats of the cosmetic composition

Preferably the composition is a scalp care composition.

The cosmetic composition may be an emulsion, lotion, cream, paste or gel. Preferably, the cosmetic composition is a scalp treatment composition, which may be a rinse-off composition or a leave-on composition. Rinse-off composition is intended to be rinsed off the scalp of the user with water after use. Leave-on composition is intended not to be rinsed off the scalp of the user immediately after use (i.e. within at least the first 2 hours, preferably at least 4 hours after application of the composition). Rinse-off compositions include shampoos and conditioners, as well as treatment compositions which can be left on scalp for from 0.5 minute to up to 2 hours, preferably from 1 minute to 1 hour, more preferably from 2 minutes to 30 minutes, still more preferably from 5 minutes to 10 minutes, prior to being rinsed off.

The preferred use of the cosmetic composition is in a shampoo. The cosmetic composition may suitably comprise from 50 to 90%, preferably from 60 to 80% water by weight of the total shampoo composition.

### Anionic cleansing surfactant

The cosmetic composition may comprise one or more anionic cleansing surfactants. The anionic cleansing surfactants refer to those which have net negative charge and can act to cleanse skin. The total level of anionic cleansing surfactant is suitably from 1 to 45%, by weight of the total composition, preferably from 10 to 25 wt%.

Preferably, the anionic cleansing surfactant is alkyl sulphate and/or ethoxylated alkyl sulphate anionic surfactant. The preferred level of such surfactant is from 0.5 to 20% by weight of the total composition, more preferably from 5 to 15 wt%.

Preferred alkyl sulphates are C₈₋₁₈ alkyl sulphate, more preferably C₁₂₋₁₈ alkyl sulphate, preferably in the form of a salt with a solubilising cation such as sodium, potassium, ammonium or substituted ammonium. Examples are sodium lauryl sulphate (SLS) or sodium dodecyl sulphate (SDS).

Preferred alkyl ether sulphates are those of formula (II):

R-O-(CH₂CH₂-O)ₙ-SO₃⁻M⁺ (II)

wherein R is a straight or branched alkyl chain having 8 to 18 (preferably 12 to 18) carbon atoms; n is the average degree of ethoxylation and ranges from 0.5 to 3 (preferably from 1 to 3); and M is a solubilizing cation such as sodium, potassium, ammonium or substituted ammonium. An example is sodium lauryl ether sulphate (SLES). The most preferred example is SLES having an average degree of ethoxylate of from 0.5 to 3, preferably from 1 to 3.

The cosmetic composition may comprise one or more further anionic surfactants which are cosmetically acceptable and suitable for topical application to skin. Examples of further anionic surfactants include alkyl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in the compositions include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate, lauric monoglyceride sodium sulphate, sodium lauryl sulphate, sodium laureth sulphate, sodium cocyl sulphate, sodium cocoyl isethionate and mixtures thereof.

The cosmetic composition may also include co-surfactants, to help impart aesthetic, physical or cleansing properties to the composition. The co-surfactant is preferably comprised in the cleansing phase of the composition. An example of a co-surfactant is a nonionic surfactant, which can be included in an amount ranging from 0.5 to 10%, preferably from 2 to 8%, more preferably from 1 to 5% by weight of the total composition.

For example, representative nonionic surfactants that can be included in the cosmetic compositions, preferably shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups. Other representative nonionic surfactants include mono- or di-alkyl alkanolamides. Examples include coco mono- or di-ethanolamide and coco monoisopropanolamide.

Further nonionic surfactants which can be included are the alkyl polyglycosides (APGs). Typically, the APG is one which comprises an alkyl group connected (optionally via a bridging group) to a block of one or more glycosyl groups. Preferred APGs are defined by the following formula (III):

R'O-(G)ₖ (III)

wherein R' is a branched or straight chain alkyl group which may be saturated or unsaturated and G is a saccharide group. R' may represent a mean alkyl chain length of from about C₅ to about C₂₀. Preferably R' represents a mean alkyl chain length of from about C₈ to about C₁₂. Most preferably the value of R' lies between about 9.5 and about 10.5. G may be selected from C₅ or C₆ monosaccharide residues and is preferably a glucoside. G may be selected from the group comprising glucose, xylose, lactose, fructose, mannose and derivatives thereof. Preferably G is glucose. The degree of polymerisation, k, may have a value of from about 1 to about 10 or more; preferably, the value of k lies from about 1.1 to about 2; most preferably the value of k lies from about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions (preferably shampoos) of the invention include the C₁₀-C₁₈ N-alkyl (C₁-C₆) polyhydroxy fatty acid amides, such as the C₁₂-C₁₈ N-methyl glucamides, as described for example in WO 92/06154 and US 5,194, 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 10 wt.%, preferably from 2 to 8, more preferably from 1 to 5 % by weight of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 22 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is an amidobetaine amphoteric surfactant of general formula (IV): 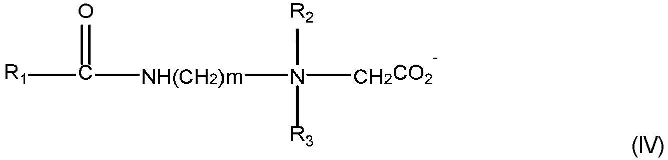 wherein m is 2 or 3; R¹C(O) is selected from linear or branched, saturated or unsaturated acyl groups having from 8 to 22 carbon atoms and mixtures thereof; and R² and R³ are each independently selected from alkyl, hydroxyalkyl or carboxyalkyl groups having from 1 to 6 carbon atoms and mixtures thereof. An example is cocamidopropyl betaine. The preferred level of such surfactant is from 0.5-10% by weight of the total composition, more preferably from 2-8 wt%, most preferably from 1-5 wt%.

A further optional but preferred surfactant is an alkyl glycinate and/or alkyl carboxyglycinate. If present, it is present at a level of from 1 to 8 wt.%, preferably 2 to 6 wt.%.

Preferably the alkyl glycinate and/or alkyl carboxyglycinate has an alkyl group of from C₈₋₂₂ carbon atoms, in the form of a salt with a solubilising cation such as sodium, potassium, ammonium or substituted ammonium. Preferred glycinates are sodium coco glycinate and sodium cocoyl glycinate.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In preferred embodiments, the cosmetic composition comprises an alkyl sulphate and/or ethoxylated alkyl sulphate anionic surfactant; and a betaine surfactant, preferably an alkyl amidopropyl betaine.

The total amount of surfactants (inclusive of any co-surfactants) in a cosmetic composition, is generally from 1 to 50 wt.%, preferably from 2 to 40 wt.%, more preferably from 10 to 25 wt.% by weight of the total composition.

### Cationic deposition polymer

The cosmetic composition may comprise at least one cationic deposition polymer. The level of the cationic polymer is suitably from 0.01 to 5%, by weight of the total composition, preferably from 0.05 to 2 wt%, more preferably from 0.1 to 1 wt%, most preferably from 0.1 to 0.5 wt%. The preferred choice of a cationic deposition polymer is a guar hydroxypropyltrimmonium chloride polymer.

The cationic deposition polymer is often used in cosmetic composition for enhancing conditioning and other sensory performance. In the present invention, it is believed that the inclusion of cationic polymer can help to enhance the delivery efficiency of piroctone olamine to the desired skin area, hence the improvement of skin natural defence.

Suitable cationic deposition polymers include cationic polygalactomannans, which are polysaccharides composed principally of galactose and mannose units and are usually found in the endosperm of leguminous seeds, such as guar, locust bean, honey locust, flame tree, and the like. Guar flour is composed mostly of a galactomannan which is essentially a straight chain mannan with single membered galactose branches. The mannose units are linked in a 1-4-β-glycosidic linkage and the galactose branching takes place by means of a 1-6 linkage on alternate mannose units. The ratio of galactose to mannose in the guar polymer is therefore one to two. Preferred cationic polygalactomannans are guar hydroxypropyltrimonium chloride polymers.

Guar hydroxypropyltrimethylammonium chlorides for use in the invention are generally comprised of a nonionic guar gum backbone that is functionalized with ether-linked 2-hydroxypropyltrimethylammonium chloride groups and are typically prepared by the reaction of guar gum with N-(3-chloro-2-hydroxypropyl) trimethylammonium chloride.

Generally, the guar hydroxypropyltrimethylammonium chlorides have an average molecular weight (weight average molecular mass (Mw) determined by size exclusion chromatography) in the range 500,000 to 3 million g/mol, more preferably 800,000 to 2.5 million g/mol.

Generally, the guar hydroxypropyltrimethylammonium chlorides have a charge density ranging from 0.5 to 1.8 meq/g. The cationic charge density of the polymer is suitably determined via the Kjeldahl method as described in the US Pharmacopoeia under chemical tests for nitrogen determination. Preferred guar hydroxypropyltrimonium chlorides have a cationic charge density from 1.1 to 1.8 meq/g. Also suitable are mixtures of guars in which one has a cationic charge density from 0.5 to 1.1 meq/g, and one has a cationic charge density from 1.1 to 1.8 meq per gram.

Guar hydroxypropyltrimethylammonium chlorides for use in the invention are commercially available from Rhodia as JAGUAR ^{®} C13S, JAGUAR ^{®} C14 and JAGUAR ^{®} C17. Also preferred is boron-free crosslinked cationic guar hydroxypropyltrimethylammonium chloride, such as the guar polymer from Lambeti as Cesmetic ^{®} BF-7.

Other suitable cationic polymers for use in the present invention include polysaccharide polymers, such as cationic cellulose derivatives and cationic starch derivatives. Preferred cationic cellulose polymers are salts of hydroxyethyl cellulose reacted with trimethyl ammonium substituted epoxide, referred to in the industry (CTFA) as polyquaternium 10 and available from Dow Chemical as UCARE^{™} Polymer JR-30M. Other suitable types of cationic cellulose include the polymeric quaternary ammonium salts of hydroxyethyl cellulose reacted with lauryl dimethyl ammonium-substituted epoxide referred to in the industry (CTFA) a Polyquaternium 24.

### Other optional components

The cosmetic composition may optionally comprise one or more components, provided that the optional components are physically and chemically compatible with the essential components described hereinbefore, and do not otherwise unduly impair sensory, formulation rheology and conditioning performance. Individual concentrations of such optional components may range from 0.001% to 10% by weight of the total composition, preferably from 0.01% to 5% wt%. Such components may include suspending agents, conditioning agents, fragrance, dyes, pigments, pH adjusting agents, pearlescers or opacifiers, viscosity modifiers, preservatives, and natural hair nutrients such as botanicals, fruit extracts, sugar derivatives and amino acids.

One of the preferred optional components is a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich. Suitable cross-linked polymers of acrylic acid and acrylate esters are Pemulen TR1 or Pemulen TR2. A suitable heteropolysaccharide gum is xanthan gum, for example that available as Kelzan mu. Mixtures of any of the above suspending agents may be used. Preferred is a mixture of cross-linked polymer of acrylic acid and crystalline long chain acyl derivative.

A most preferred example is a crosslinked polyacrylate polymer.

Suspending agent, if included, will generally be present in the composition at a level of from 0.01 to 5 wt.%, preferably from 0.1 to 2.5 wt.%, more preferably from 0.25 to 1 wt.%.

Another preferred optional component is a conditioning agent, providing conditioning benefit. Typically, the most popular conditioning agents used in cosmetic compositions are water-insoluble oily materials such as mineral oils, naturally occurring oils such as triglycerides and silicone oils. Conditioning benefit is achieved by the oily material being deposited onto the skin resulting in the formation of a film, which makes the skin more lubricant and less dry. Preferably, the conditioning agent is non-volatile, meaning that it has a vapour pressure of less than 1000 Pa at 25 °C.

Preferably, the composition comprises discrete dispersed droplets of a water-insoluble conditioning agent, which has a mean droplet diameter (D_{3,2}) of less than 15 microns, preferably less than 10 microns, more preferably less than 5 microns, most preferably less than 3 microns. The mean droplet diameter (D_{3,2}) of a water-insoluble conditioning agent may be measured by means of a laser light scattering technique, for example using a 2600D Particle Sizer from Malvem Instruments.

The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of cross-linking, as are described for example in WO 96/31188. Preferably, the silicone oil comprises dimethicone, dimethiconol or a mixture thereof.

The viscosity of the emulsified silicone itself (not the emulsion or the final hair care composition) is typically at least 10,000 cSt (centi-Stokes=mm^{2.}S⁻¹) at 25 °C, preferably at least 60,000 cSt, most preferably at least 500,000 cSt, ideally at least 1,000,000 cSt. Preferably the viscosity does not exceed 10⁹ cSt for ease of formulation. Suitable methods for measuring the kinematic viscosity of silicone oils are known to those skilled in the art, e.g. capillary viscometers. For high viscosity silicones, a constant stress rheometer can be used to measure viscosity.

Suitable emulsified silicones for use in the compositions are available as pre-formed silicone emulsions from suppliers of silicones such as Dow Coming and GE silicones. The use of such pre-formed silicone emulsion is preferred for ease of processing and control of silicone particle size. Such pre-formed silicone emulsions will typically additionally comprise a suitable emulsifier and may be prepared by a chemical emulsification process such as emulsion polymerisation, or by mechanical emulsification using a high shear mixer.

Examples of suitable pre-formed silicone emulsions include DC1785, DC1788, DC7128, all available from Dow Coming. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

Preferably, silicone emulsion droplets are blended with certain types of surface active block polymers of a high molecular weight to form silicone emulsions, as described for example in WO03/094874. One preferred form of the surface active block polymer having polyoxypropylene and polyoxyethylene groups as the hydrophobic and hydrophilic part respectively has formula V and has the CTFA designation poloxamer, known commercially under the trade name "Pluronic" from BASF.

V) HO(CH₂CH₂O)ₓ(CH(CH₃)CH₂O)_{y}(CH₂CH₂O)ₓ H

Suitably, the mean value of x in formula I is 4 or more, preferably 8 or more, more preferably 25 or more, yet more preferably 50 or more and most preferably 80 or more. The mean value of x is typically no greater than 200. Suitably, the mean value of y is 25 or more, preferably 35 or more, more preferably 45 or more and most preferably 60 or more. The mean value of y is typically no greater than 100.

Another preferred form of the surface active block polymer is according to formula VI and has the CTFA designation Poloxamine. Those are commercially available under the trade name "Tetronic" from BASF.

VI) (HO(CH₂CH₂O)ₐ(CH(CH₃)CH₂O)_{b})₂-N-CH₂-CH₂-N-((OCH₂CH(CH₃))_{b}(OCH₂CH₂)ₐOH)₂

Suitably, the mean value of a is 2 or more, preferably 4 or more, more preferably 8 or more, even more preferably 25 or more and most preferably 40 or more. The mean value of a is typically no greater than 200. The mean value of b is suitably 6 or more, preferably 9 or more, more preferably 11 or more and most preferably 15 or more. The mean value of b is typically no greater than 50.

Preferably, the surface active block polymer is poloxamer and/or poloxamine, more preferably, the surface active block polymer is poloxamer.

Preferably, the surface active block polymer is blended with dimethicone. The weight ratio of dimethicone to surface active block polymer in the blend is preferably in the range from 2:1 to 200:1, more preferably from 5:1 to 50:1, even more preferably from 10:1 to 40:1, most preferably from 15:1 to 30:1.

The water-insoluble conditioning agent is generally present in the composition in an amount from 0.05 to 15%, preferably from 0.1 to 10%, more preferably from 0.5 to 8%, most preferably from 1 to 5%, based on the total weight of the composition and including all ranges subsumed therein.

The composition may preferably comprise a peariescer. The preferred pearlescer is ethylene glycol ester as disclosed in US4885107, incorporated herein by reference. Preferably, the ethylene glycol ester is a mono- or, di-ester of glycol, more preferably a di-ester of glycol.

Preferably ethylene glycol mono- or di-ester is an ethylene glycol mono- or di-ester of a C₁₂₋₂₂ fatty acid, more preferably an ethylene glycol mono- or di-ester of a saturated C₁₂₋₂₂ fatty acid. Most preferred are the diesters comprising a mixture of palmitate and stearate. The amount of stearate should be in the range of about 10% to about 42% or in the range of about 55% to about 80% with palmitate accounting for the remainder. The amount of stearate is preferably from about 60% to about 75%, more preferably from about 80-95%, most preferably 100%. The most suitable example of a pearlescer is an ethylene glycol distearate. Pearlescer may also perform the function as a suspending agent.

The level of the ethylene glycol ester can be suitably from about 0.5% to about 6%, preferably from about 1 % to about 4%, by weight of the total composition.

The viscosity of the composition suitably ranges from 3,000 to 10,000 mPa.s, preferably from 4,000 to 8,000 mPa.s, more preferably from 5,000 to 7,000 mPa.s when measured using a Brookfield V2 viscometer (spindle RTV5, 1 minute, 20rpm) at 30°C.

The pH of the composition of the invention preferably ranges from 3 to 9, more preferably from 5 to 7, still more preferably from 5.5 to 6.5.

The present invention further provides piroctone olamine for use in a method for the treatment of increasing natural defence of skin.

The present invention is illustrated by means of the following examples.

### EXAMPLES

In the context of the present invention enhancement means a change in level of gene when compared to a baseline level of biomarker.

### Example 1

Primary human epidermal keratinocytes were obtained from Lonza. Cells were cultured in monolayer in KGM-Gold medium. For the experiment keratinocytes were differentiated by incubating for five days in KGM-Gold medium supplemented with 1.2 mM calcium chloride. Piroctone olamine was supplied by Sigma. Differentiated keratinocyte cultures were dosed with 7.5 µM piroctone olamine and 0.1% ethanol (Group 1, n=4), or with 0.1% ethanol only (Group 2, n=4) and incubated for 24 hours. Cells were then lysed in RLT buffer and subjected to RNA extraction using Qiagen RNeasy Micro Kit according to manufacturer's instructions. The levels of gene expression were determined using Agilent SurePrint G3 Human Gene Expression 8x60k v3 Microarrays in conjunction with Agilent SureHyb hybridisation and SureScan High-Resolution scanning technology, in accordance with manufacturer's protocol. Microarray data processing and statistical analysis was carried out in Agilent GeneSpring GX14 software. To identify differentially expressed genes in piroctone olamine treated cultures (Group 1) compared to vehicle control cultures (Group 2), t-test was applied (p≤0.05) with or without Benjamini-Hochberg false discovery rate correction (FDR≤0.05). Table 1 shows a clear increase in gene expression of epidermal barrier biomarkers in differentiated keratinocytes treated with piroctone olamine, compared to control.

**Table 1.**

| **Fold change gene expression changes in piroctone olamine dosed differentiated keratinocytes compared to control.** | | | | |
|---|---|---|---|---|
| **Function** | **Genes** | **Gene Symbol** | **Fold Change (Group 1/Group 2)** | **p value** |
| **Corneodesmosome formation** | **Desmocolin 2** | **DSC2** | 2.49 | 0.00012 |
| | **Desmoglein 4** | **DSG4** | 2.44 | 0.00012 |
| | **Plakophilin 1** | **PKP1** | 1.75 | 0.00010 |
| | **Corneodesmosin** | **CDSN** | 1.20 | 0.24142* |
| **Tight junction formation** | **Claudin 17** | **CLDN17** | 2.99 | 0.00017 |
| | **Claudin 25** | **CLDN25** | 2.05 | 0.00112 |
| | **Claudin 4** | **CLDN4** | 1.76 | 0.00171 |
| | **Claudin 7** | **CLDN7** | 1.65 | 0.00114 |
| | **Zona Occludens 1** | **ZO1 (TJP1)** | 1.3 | 0.11304* |
| **Cornified envelop formation** | **Late comified envelope 3A** | **LCE3A** | 2.58 | 0.00099 |
| | **Late comified envelope 3C** | **LCE3C** | 2.71 | 0.00024 |
| | **Late comified envelope 3B** | **LCE3B** | 1.67 | 0.00759 |
| | **Small proline-rich protein 2D** | **SPRR2D** | 1.52 | 0.00169 |
| | **Involucrin** | **IVL** | 1.40 | 0.0063 |

| | | | | |
|---|---|---|---|---|
| *not significant | | | | |

The results demonstrate that fold change gene expression changes in piroctone olamine dosed differentiated keratinocytes compared to control, thus demonstrating the advantage.

The higher the fold change, the more effective the mRNA biomarker/mRNA biomarker group is

### Example 2

Human ex vivo skin model production and piroctone olamine treatment were conducted by Genoskin (France). NativeSkin models of 15 mm diameter ex *vivo* human skin explants with silicon rings were produced from three donors and cultured in NativeSkin culture medium under 5% CO₂ humidified atmosphere in 37 °C. Skin explants were treated topically with 50 ul of solution containing 30 uM piroctone olamine in PBS with 1% ethanol (Group 1, n=3), or control PBS solution containing 1% ethanol (Group 2, n=3), and were incubated for 24 hours. Following one day of topical treatment, piroctone olamine and control solutions were removed using cotton swabs and each model was unmolded from silicon ring and re-punch at 12 mm diameter. Half of each skin biopsy was processed for RNA using OMNI-TH Rotor Stator tissue homogeniser and Qiagen RNeasy Fibrous Tissue RNA Mini Kit according to manufacturer's instructions. RNA sequencing was used for gene expression analysis. Lexogen QuantSeq3' FWD libraries were prepared from RNA samples and sequenced via single read (1x75bp) performed using NextSeq 500/550 Mid-Output Kit on the NextSeq 500 Illumina platform. FASTQ data files were then uploaded to the BlueBee genomic platform and read-trimming, alignment, and read count were performed using QuantSeq data processing pipeline. Differential gene expression analysis for Group 1/Group 2 comparison was run in Bluebee genomic platform using QuantSeq DE pipeline (FDR corrected p≤0.05). Table 2 shows an increase in gene expression of epidermal barrier biomarkers in human skin explants treated topically with piroctone olamine (Group 1) compared to control (Group 2).

**Table 2.**

| **Function** | **Genes** | **Gene Symbol** | **Fold Change (Group 1/Group 2)** | **corrected p value** |
|---|---|---|---|---|
| **Tight junction formation** | **Claudin 1** | **CLDN 1** | 2.1 | 0.01 |
| | **Zona Occludens 2** | **ZO2 (TJP2)** | 3.5 | 1.17E-06 |
| **Cornified envelop formation** | **Involucrin** | **IVL** | 2.7 | 0.01 |

## Claims

1. A non-therapeutic method of strengthening a skin barrier by application of a composition comprising at least one mRNA biomarker and/or mRNA biomarker group enhancement compound in which the mRNA biomarker/mRNA biomarker group is involved in cornified envelope formation, wherein the composition comprises a piroctone compound.

2. A method according to claim 1 wherein the piroctone compound is present at a level of 0.3 to 1.0 wt% of the composition.

3. A method according to claim 1 or claim 2 in which the mRNA biomarker group is further involved in comeodesmosome formation and tight junction formation.

4. A method according to any preceding claim in which the skin barrier is the skin barrier of the scalp.

5. A method according to any preceding claim in which the mRNA biomarker is selected from the group consisting of Desmocolin 2,Desmoglein 4, Plakophilin 1, Claudin 4 Claudin 7 Claudin 17 Claudin 25 Late cornified envelope 3A Late cornified envelope 3C Late cornified envelope 3B, Small proline-rich protein 2D, Claudin 1, Zona Occludens 2 and Involucrin.

6. A method according to any preceding claim wherein the composition is a scalp care composition.

7. A method according to any preceding claims wherein the mRNA biomarker/mRNA biomarker group is selected from the group consisting of anti-dandruff actives, anti-microbial actives, anti-fungal actives and mixtures thereof.

8. A method according to any preceding claims wherein the mRNA biomarker/mRNA biomarker group is piroctone olamine.

9. A method according to any preceding claim in which the composition further comprises an anionic surfactant.

10. A method according to any preceding claim in which the composition further comprised a conditioning agent.

11. A composition comprising at least one mRNA biomarker enhancement compound in which the mRNA biomarker enhancement compound is involved in cornified envelope formation for use as a medicament in strengthening the skin barrier, wherein the composition comprises a piroctone compound.

12. The cosmetic use for strengthening a skin barrier of a composition comprising at least one mRNA biomarker enhancement compound in which the mRNA biomarker is involved in cornified envelope formation, wherein the composition comprises a piroctone compound.

## Patentansprüche

1. Nicht-therapeutisches Verfahren zur Stärkung einer Hautbarriere unter Verwendung einer Zusammensetzung, die mindestens eine mRNA-Biomarker- und/oder eine mRNA-Biomarkergruppen-Verstärkungsverbindung umfasst, wobei der mRNA-Biomarker/die mRNA-Biomarkergruppe an der Bildung einer verhornten Hülle beteiligt ist, wobei die Zusammensetzung eine Piroctonverbindung umfasst.

2. Verfahren nach Anspruch 1, wobei die Piroctonverbindung in einer Menge von 0,3 bis 1,0 Gew.-% der Zusammensetzung vorhanden ist.

3. Verfahren nach Anspruch 1 oder Anspruch 2, wobei die mRNA-Biomarkergruppe außerdem an der Bildung von Corneodesmosomen und der Tight-Junction-Bildung beteiligt ist.

4. Verfahren nach einem vorhergehenden Anspruch, wobei die Hautbarriere die Hautbarriere der Kopfhaut ist.

5. Verfahren nach einem vorhergehenden Anspruch, wobei der mRNA-Biomarker aus der Gruppe ausgewählt ist, bestehend aus Desmocolin 2, Desmoglein 4, Plakophilin 1, Claudin 4, Claudin 7, Claudin 17, Claudin 25, spät-verhornter Hülle 3A, spät-verhornter Hülle 3C, spät-verhornter Hülle 3B, kleinem prolinreichen Protein 2D, Claudin 1, Zona Occludens 2 und Involucrin.

6. Verfahren nach einem vorhergehenden Anspruch, wobei die Zusammensetzung eine Zusammensetzung zur Kopfhautpflege ist.

7. Verfahren nach irgendeinem der vorhergehenden Ansprüche, wobei der mRNA-Biomarker/die mRNA-Biomarkergruppe aus der Gruppe, bestehend aus Antischuppenwirkstoffen, antimikrobiellen Wirkstoffen, Antipilzwirkstoffen und Mischungen davon, ausgewählt ist.

8. Verfahren nach einem der vorhergehenden Ansprüche, wobei der mRNA-Biomarker/die mRNa-Biomarkergruppe ein Piroctonolamin ist.

9. Verfahren nach einem vorhergehenden Anspruch, wobei die Zusammensetzung außerdem ein anionisches Tensid umfasst.

10. Verfahren nach einem vorhergehenden Anspruch, wobei die Zusammensetzung außerdem ein Konditionierungsmittel umfasst.

11. Zusammensetzung, umfassend mindestens eine mRNA-Biomarker-Verstärkerverbindung umfasst, wobei die mRNA-Biomarker-Verstärkerverbindung an der Bildung einer verhornten Hülle zur Verwendung als Medikament zur Stärkung der Hautbarriere beteiligt ist, wobei die Zusammensetzung eine Piroctonverbindung umfasst.

12. Kosmetische Verwendung einer Zusammensetzung zur Stärkung einer Hautbarriere, umfassend mindestens eine mRNA-Biomarker-Verstärkerverbindung, wobei der mRNA-Biomarker an der Bildung einer verhornten Hülle beteiligt ist, wobei die Zusammensetzung eine Piroctonverbindung umfasst.

## Revendications

1. Méthode non thérapeutique de renforcement de la barrière cutanée par application d'une composition comprenant au moins un composé amplifiant un biomarqueur ARNm et/ou un groupe de biomarqueurs ARNm, dans laquelle le biomarqueur ARNm/groupe de biomarqueurs ARNm est impliqué dans la formation d'enveloppe cornifiée, dans laquelle la composition comprend un composé de piroctone.

2. Méthode selon la revendication 1, dans laquelle le composé de piroctone est présent à raison de 0,3 à 1,0 % en poids de la composition.

3. Méthode selon la revendication 1 ou la revendication 2, dans laquelle le groupe de biomarqueurs ARNm est en outre impliqué dans la formation de cornéodésmosomes et la formation de jonctions étanches.

4. Méthode selon l'une quelconque des revendications précédentes, dans laquelle la barrière cutanée est la barrière cutanée du cuir chevelu.

5. Méthode selon l'une quelconque des revendications précédentes, dans laquelle le biomarqueur ARNm est choisi dans le groupe constitué par la desmocoline 2, la desmogléine 4, la plakophiline 1, la claudine 4, la claudine 7, la claudine 17, la claudine 25, l'enveloppe cornifiée tardive 3A, l'enveloppe cornifiée tardive 3C, l'enveloppe cornifiée tardive 3B, la petite protéine riche en proline 2D, la claudine 1, Zona Occludens 2, et l'involucrine.

6. Méthode selon l'une quelconque des revendications précédentes, dans laquelle la composition est une composition de soin du cuir chevelu.

7. Méthode selon l'une quelconque des revendications précédentes, dans laquelle le biomarqueur ARNm/groupe de biomarqueurs ARNm est choisi dans le groupe constitué par les principes actifs antipelliculaires, les principes actifs antimicrobiens, les principes actifs antifongiques, et leurs mélanges.

8. Méthode selon l'une quelconque des revendications précédentes, dans laquelle le biomarqueur ARNm/groupe de biomarqueurs ARNm est la piroctone olamine.

9. Méthode selon l'une quelconque des revendications précédentes, dans laquelle la composition comprend en outre un tensioactif anionique.

10. Méthode selon l'une quelconque des revendications précédentes, dans laquelle la composition comprend en outre un agent conditionneur.

11. Composition comprenant au moins un composé amplifiant un biomarqueur ARNm, dans laquelle le composé amplifiant un biomarqueur ARNm est impliqué dans la formation d'enveloppe cornifiée, pour une utilisation en tant que médicament dans le renforcement de la barrière cutanée, laquelle composition comprend un composé de piroctone.

12. Utilisation cosmétique, pour renforcer une barrière cutanée, d'une composition comprenant au moins un composé amplifiant un biomarqueur ARNm, dans laquelle le composé amplifiant un biomarqueur ARNm est impliqué dans la formation d'enveloppe cornifiée, dans laquelle la composition comprend un composé de piroctone.
